# EUROPÄISCHE PATENTSCHRIFT

(11) **EP 1 287 356 B1**
(45) Veröffentlichungstag und Bekanntmachung des Hinweises auf die Patenterteilung: **17.03.2004**
(21) Anmeldenummer: 01938242.3
(22) Anmeldetag: 29.05.2001
(51) Int. Cl.: G01N 33/52, G01N 33/497

(54) **VERFAHREN ZUM NACHWEIS VON ALPHA-OXOALDEHYDEN IN VOLLBLUT, BLUTPLASMA UND/ODER SERUM EINES PATIENTEN**
METHOD FOR DETECTING ALPHA-OXOALDEHYDES IN THE WHOLE BLOOD, BLOOD PLASMA AND/OR SERUM OF A PATIENT
PROCEDE POUR DETECTER LA PRESENCE D'ALPHA-OXO-ALDEHYDES DANS LE SANG TOTAL, LE PLASMA SANGUIN ET/OU LE SERUM D'UN PATIENT

(30) Priorität: 09.06.2000 DE 10028548
(43) Veröffentlichungstag der Anmeldung: 05.03.2003
(73) Patentinhaber: INSTITUT FÜR CHEMO- UND BIOSENSORIK Münster e.V., 48149 Münster (DE)
(72) Erfinder: KLEIBÖHMER, Wolfgang, 48155 Münster (DE); SCHULZE-PELLENGAHR, Uta, 59387 Ascheberg (DE)
(74) Vertreter: Pfenning, Meinig & Partner
(86) Internationale Anmeldenummer: PCT/EP2001/006101
(87) Internationale Veröffentlichungsnummer: WO 2001/094942

(56) Entgegenhaltungen:
- WO-A-99/56790
- US-A- 5 770 028
- KALAPOS MIKLOS PETER: "Methylglyoxal in living organisms: Chemistry, biochemistry, toxicology and biological implications." TOXICOLOGY LETTERS (SHANNON)., Bd. 110, Nr. 3, 22. November 1999 (1999-11-22), Seiten 145-175, XP002175623 ISSN: 0378-4274
- THORNALLEY PAUL J ET AL: "Formation of glyoxal, methylglyoxal and 3-deoxyglucosone in the glycation of proteins by glucose." BIOCHEMICAL JOURNAL, Bd. 344, Nr. 1, 15. November 1999 (1999-11-15), Seiten 109-116, XP002175624 ISSN: 0264-6021
- DATABASE BIOSIS [Online] BIOSCIENCES INFORMATION SERVICE, PHILADELPHIA, PA, US; 1995 LIN YUMEI ET AL: "Protocol for collection and HPLC analysis of volatile carbonyl compounds in breath." Database accession no. PREV199598370397 XP002175625 & CLINICAL CHEMISTRY, Bd. 41, Nr. 7, 1995, Seiten 1028-1032, ISSN: 0009-9147
- DATABASE BIOSIS [Online] BIOSCIENCES INFORMATION SERVICE, PHILADELPHIA, PA, US; 1980 STOWELL A R ET AL: "BREATH AND BLOOD ACETALDEHYDE CONCENTRATIONS AND THEIR CORRELATION DURING NORMAL AND CALCIUM CARBIMIDE MODIFIED ETHANOL OXIDATION IN MAN" Database accession no. PREV198070006603 XP002175626 & BIOCHEMICAL PHARMACOLOGY, Bd. 29, Nr. 5, 1980, Seiten 783-788, ISSN: 0006-2952
- DATABASE BIOSIS [Online] BIOSCIENCES INFORMATION SERVICE, PHILADELPHIA, PA, US; 1980 YADIN H: "AEROSOL VACCINATION AGAINST NEWCASTLE DISEASE VIRUS INHALATION AND RETENTION DURING VACCINATION" Database accession no. PREV198070051000 XP002175627 & AVIAN PATHOLOGY, Bd. 9, Nr. 2, 1980, Seiten 163-170, ISSN: 0307-9457
- DATABASE BIOSIS [Online] BIOSCIENCES INFORMATION SERVICE, PHILADELPHIA, PA, US; 1988 SHANE B S ET AL: "COMPARATIVE MUTAGENICITY OF NINE BRANDS OF COFFEE TO SALMONELLA-TYPHIMURIUM TA100 TA102 AND TA104" Database accession no. PREV198885121681 XP002175690 & ENVIRONMENTAL AND MOLECULAR MUTAGENESIS, Bd. 11, Nr. 2, 1988, Seiten 195-206, ISSN: 0893-6692
- DATABASE BIOSIS [Online] BIOSCIENCES INFORMATION SERVICE, PHILADELPHIA, PA, US; 1995 MISRA KANIKA ET AL: "Glyoxalase III from Escherichia coli: A single novel enzyme for the conversion of methylglyoxal into D-lactate without reduced glutathione." Database accession no. PREV199598176476 XP002175628 & BIOCHEMICAL JOURNAL, Bd. 305, Nr. 3, 1995, Seiten 999-1003, ISSN: 0264-6021
- DATABASE BIOSIS [Online] BIOSCIENCES INFORMATION SERVICE, PHILADELPHIA, PA, US; 1990 MUNGER J W ET AL: "FOGWATER CHEMISTRY AT RIVERSIDE CALIFORNIA USA" Database accession no. PREV199090080547 XP002175629 & ATMOSPHERIC ENVIRONMENT PART B URBAN ATMOSPHERE, Bd. 24, Nr. 2, 1990, Seiten 185-206, ISSN: 0957-1272

## Beschreibung

Die vorliegende Erfindung bezieht sich auf ein Verfahren zum Nachweis von α-Oxoaldehyden in Vollblut, im Blutplasma und/oder im Serum von Patienten.

Infolge von langjährigen Diabetes-Erkrankungen, d.h. insulinabhängiger und/oder insulinunabhängiger Diabetes, auftretende Komplikationen, wie Nierenschädigungen oder Trübungen der Augenlinsen lassen sich nur schwer und oft erst spät erkennen. Mitverantwortlich für diese Komplikationen und Folgeerkrankungen sind oftmals reaktive Stoffwechselprodukte, die mit Kollagen, Enzymen oder anderen zellulären Bestandteilen Glykolate bilden und durch diesen Mechanismus als Zellgifte wirken können. Entsprechende Marker für diese Erkrankung sind nach dem Stand der Technik nur über eine aufwendige Blutanalytik im Labor erfaßbar.

In den letzten drei bis vier Jahren haben Untersuchungen zur Aufklärung von Folgeerkrankungen im Verlauf einer langjährigen Diabetes gezeigt, daß die cytotoxischen α-Oxoaldehyde eine wichtige und ursächliche Rolle spielen (Beisswenger et al. (1999) Diabetes, Band 48, S. 198-202). Daher wird in diesem Zusammenhang insbesondere die Rolle der α-Oxoaldehyde wie Methylglyoxal, Glyoxal oder 3-Deoxyglucuron diskutiert. Diese Stoffe werden in den roten Blutkörperchen gebildet und liegen in sehr geringen Konzentrationen im Vollblut, im Blutplasma und im Serum vor.

Von besonderem Interesse ist hierbei das α-Oxoaldehyd Methylglyoxal. Methylglyoxal kann aus Triosephosphat, der Metabolisierung von Keton-Körpern sowie bei der Verstoffwechselung von Threonin entstehen und wird durch Glyoxalase weiter abgebaut. Methylglyoxal reagiert dann mit Proteinen unter Bildung von Imidazolon-Derivaten und bis-Lysyl-Quervernetzungen. Diese Quervernetzung der Proteine kann zur Stabilisierung von Kollagen und damit zur Verdickung von Membranen führen. Damit erklären diese Reaktionen zumindest einen Teil der diabetischen Komplikationen, wie Nierenversagen und Linsentrübung.

Die alleinige Blutzuckerbestimmung, wie sie bisher bei Diabetikern üblich ist, gibt keinen direkten Rückschluß auf die momentane Methylglyoxalkonzentration im Vollblut, im Plasma oder im Serum.

Nach dem Stand der Technik erfolgt die Bestimmung von α-Oxoaldehyden daher ausschließlich invasiv, d.h. aus dem Blutplasma. Bei Patienten mit einer insulinabhängigen Diabetes konnte von Thornally T.J., "Advanced Glycation and the development of diabetic complications. Unifying the involvement of glucose, methylglyoxal and oxidative stress" Endocrinology and Metabolism, 1996, 3, 149-166, direkt im Plasma im Vergleich zu gesunden Vergleichsprobanden eine fünf- bis sechsfach höhere, bei Patienten mit insulinabhängiger Diabetes eine zwei- bis dreifach höhere Methylglyoxalkonzentration nachgewiesen werden. Alternativ wäre eine Bestimmung von α-Oxoaldehyd im Urin möglich, ist jedoch in der Literatur nicht beschrieben, da die zu erwartenden physiologisch bedingt sehr niedrigen Konzentrationen im Urin einen zu hohen Analyseaufwand erfordern würden. Daher ist eine direkte Bestimmung der α-Oxoaldehyde im Plasma erforderlich.

Aufgrund dieser Untersuchungsmethoden nach dem Stand der Technik sind regelmäßige Kontrollen und eine eventuelle bedarfsorientierte Dosierung von Medikamenten zur gezielten Reduzierung von α-Oxoaldehyden wie Methylglyoxal im Vollblut, Blutplasma und Serum sehr aufwendig.

Die WO 98/57 145 A1 beschreibt ein Verfahren zur frühzeitigen Erkennung von biologischen Zuständen wie Krankheiten durch die Analyse von geeigneten gasförmigen Proben.

Die WO 99/56 790 A2 beschreibt ein diagnostisches Kit zur Durchführung von Atemtests, die für die Diabetikdiagnose verwendet werden.

Die DD 219 287 A1 beschreibt ein Meßgerät zur Kontrolle von Blutzucker bei Diabetikern auf Basis der selektiven Bestimmung der Konzentration von Aceton im Atemgas auf der Grundlage der Strahlungsabsorption unter Verwendung einer Meßküvette, von Filtern, Linsen und einem Stromversorgungsteil.

Diese Verfahren nach dem Stand der Technik sind folglich extrem aufwendig und kostenträchtig und eignen sich daher bisher nicht für eine regelmäßige Routinekontrolle zur Bestimmung des α-Oxoaldehydgehaltes im Vollblut, Blutplasma oder Serum.

KALAPOS MIKLOS PETER: 'Methylglyoxal in living organisms: Chemistry, biochemistry, toxicology and biological implications.' TOXICOLOGY LETTERS (SHANNON)., Bd. 110, Nr. 3, 22. November 1999 (1999-11-22), Seiten 145-175, XP002175623 ISSN: 0378-4274, offenbart Verfahren zur Bestimmung von Methylglyoxal.

In den zwei folgenden Dokumenten werden Aldehyde, u.a. Acetaldehyd, im Atem von Patienten gemessen,

DATABASE BIOSIS [Online] BIOSCIENCES INFORMATION SERVICE, PHILADELPHIA, PA, US; 1995 LIN YUMEI ET AL: 'Protocol for collection and HPLC analysis of volatile carbonyl compounds in breath.' Database accession no. PREV199598370397 XP002175625 & CLINICAL CHEMISTRY, Bd. 41, Nr. 7, 1995, Seiten 1028-1032, ISSN: 0009-9147,

DATABASE BIOSIS [Online] BIOSCIENCES INFORMATION SERVICE, PHILADELPHIA, PA, US; 1980 STOWELL A R ET AL: 'BREATH AND BLOOD ACETALDEHYDE CONCENTRATIONS AND THEIR CORRELATION DURING NORMAL AND CALCIUM CARBIMIDE MODIFIED ETHANOL OXIDATION IN MAN' Database accession no. PREV198070006603 XP002175626 & BIOCHEMICAL PHARMACOLOGY, Bd. 29, Nr. 5, 1980, Seiten 783-788, ISSN: 0006-2952,
wobei das erste Dokument verschiedene Aldehyde bzw. Ketone beschreibt und dann deren Auftreten im Atem untersucht; und im zweiten Dokument die Korrelation zwischen Blut und Atemwerten von Acetaldehyd dargestellt wird.

Aufgabe der vorliegenden Erfindung ist es daher, ein Verfahren zum Nachweis von α-Oxoaldehyden im Vollblut, Blutplasma und/oder Serum eines Patienten zur Verfügung zu stellen, das einfach, kostengünstig und sicher durchgeführt werden kann.

Diese Aufgabe wird durch ein Verfahren mit den Merkmalen des Anspruches 1 gelöst. Vorteilhafte Weiterbildungen des erfindungsgemäßen Verfahrens werden in den abhängigen Ansprüchen gegeben.

Das erfindungsgemäße Verfahren beruht darauf, daß nicht etwa eine Untersuchung am Patienten selbst durchgeführt wird, sondern lediglich eine Atemluftprobe (also Atemgas und/oder Atemkondensat) des Patienten untersucht wird. Mit diesem Verfahren werden, α-Oxoaldehyde auch in der Atemluft nachgewiesen. Offenbar sind α-Oxoaldehyde, die hauptsächlich im Blutplasma vorliegen, zusätzlich leicht membrangängig, so daß diese im Atemgas oder im Atemkondensat nachgewiesen werden können. Weiterhin sind sie im Gegensatz zu anderen bekannten Markern wie Aceton, stoffwechselspezifisch und in Luft nicht ubiquitär auftretend. Damit ist eine einfache, kostengünstige und sichere, regelmäßige und nicht invasive Überwachung der α-Oxoaldehydbildung im Vollblut, Blutplasma und Serum bei Diabetikern möglich, so daß durch geeignete Medikation das Risiko möglicher Folgeerkrankungen von Diabetes reduziert werden kann. Damit besteht die Möglichkeit bei Patienten mit Langzeit-Diabetes neue

Strategien zur Vorbeugung von diabetischen Komplikationen einzuführen. Zum Beispiel ist es möglich, daß durch Verabreichung von sogenannten Scavenger-Verbindungen die Konzentration von α-Oxoaldehyden im Blut reduziert werden kann. Derartige Therapien werden durch das erfindungsgemäße nichtinvasive Testverfahren unterstützt und erst routinemäßig möglich.

Die Probennahme kann entweder durch das Aufblasen entsprechender Probenahmebeutel und/oder geeigneter Probenbehältnisse oder durch das Durchleiten von Atemluft durch einen Impinger erfolgen. Dieser Irnpinger kann mit einem Derivatisierungsmittel zum direkten Nachweis (durch Fluoreszenz oder photometrisch) oder für eine chromatographische Bestimmung gefüllt sein. Möglich ist auch die Befüllung der Impinger mit Reagenzien zur elektrochemischen, spektrometrischen (z.B. IR-spektrometrischen), enzymatischen oder immunchemischen Detektion.

Ein Nachweisverfahren besteht in der enzymatischen Umsetzung von Methylglyoxal mit Glyoxalase zu Lactat und die anschließende Bestimmung von Lactat mit einem modifizierten Sensor.

Wesentlich bei dem vorliegenden Verfahren ist dabei, daß sämtliche Bestandteile einer Atemluftprobe, d.h. sowohl die Atemluft als auch das Atemkondensat sich zur Analyse des α-Oxoaldehydgehaltes eignen.

Im folgenden wird ein Beispiel für das erfindungsgemäße Verfahren beschrieben.

Bei diesem Beispiel wurde eine Atemluftprobe einer Patientin mit Diabetes vom Typ II genommen und auf die α-Oxoaldehyde Glyoxal und Methylglyoxal untersucht. Als Untersuchungsverfahren wurde die DNPH-Methode gemäß Zurek et al., Analyst 1999, Bd. 124, S. 1291-1295 verwendet.

Die Probenahme der Atemluft wurde wie folgt durchgeführt: Über ein Mundstück mit Schlauch wurde von der Testperson/ dem Patienten ein Probenahmebeutel (hier ein Tedlar®-Beutel) mit einem Volumen von 30 Litern aufgeblasen.

Die Probenahme über einen Tedlar-Beutel wurde durchgeführt, um anschließend ein definiertes Volumen über die Sammelphase leiten zu können.

Im Anschluss an die Probenahme wurde mit Hilfe einer Pumpe ein definiertes Luftvolumen (20 L bei einem Volumenstrom von < 2 l/min) aus dem Beutel über eine Kartusche, die das Sammelmaterial enthält, gesaugt. Die kommerziell erhältliche Kartusche war mit 2,4-Dinitrophenylhydrazin belegtem Kieselgel (Supelco: Artikelnummer 505358) als Sammelmaterial befüllt. Die α-Oxoaldehyde reagierten entsprechend der folgenden Gleichung zu 2,4-Dinitrohydrazonen und wurden auf dem Kieselgel zurückgehalten.

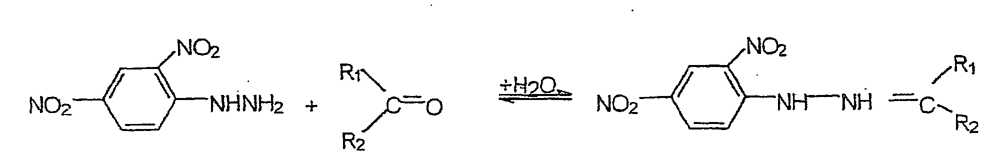

Die entstandenen Derivate wurden anschließend mit 4ml Acetonitril von der Kartusche eluiert und das erhaltene Eluat bis zur Trockne eingeengt. Der verbleibende Rückstand wurde mit 500 µl Acetonitril wieder aufgelöst. Ein Aliquot von 20 µl wurde in das HPLC-System zur Analyse überführt.

Zur Analyse wurde ein HPLC-Verfahren nach dem Prinzip der Umkehrphasenchromatographie mit folgende methodischen Parametern verwendet:
**Säule**: Hypersil BDS C18, Säulenlänge: 10 cm, Säulendurchmesser: 4mm, Teilchengrösse: 3µm
**Mobile Phase**: Gemisch aus Acetonitril und Wasser, Volumenstrom von 0,5 ml/min
Startbedingungen: Gemisch aus Acetonitril und Wasser (50:50;Volumenverhältnisse)
Gradient: Konstante Startbedingungen über 10 Minuten, dann innerhalb von 27 Minuten auf einen Volumenanteil Acetonitril von 100%. Die Daten wurden mittels UV-Detektion bei 360 und 404 nm erfaßt und mit einer Hewlett-Packard Chem-Station aufgezeichnet.

### Figur 1:

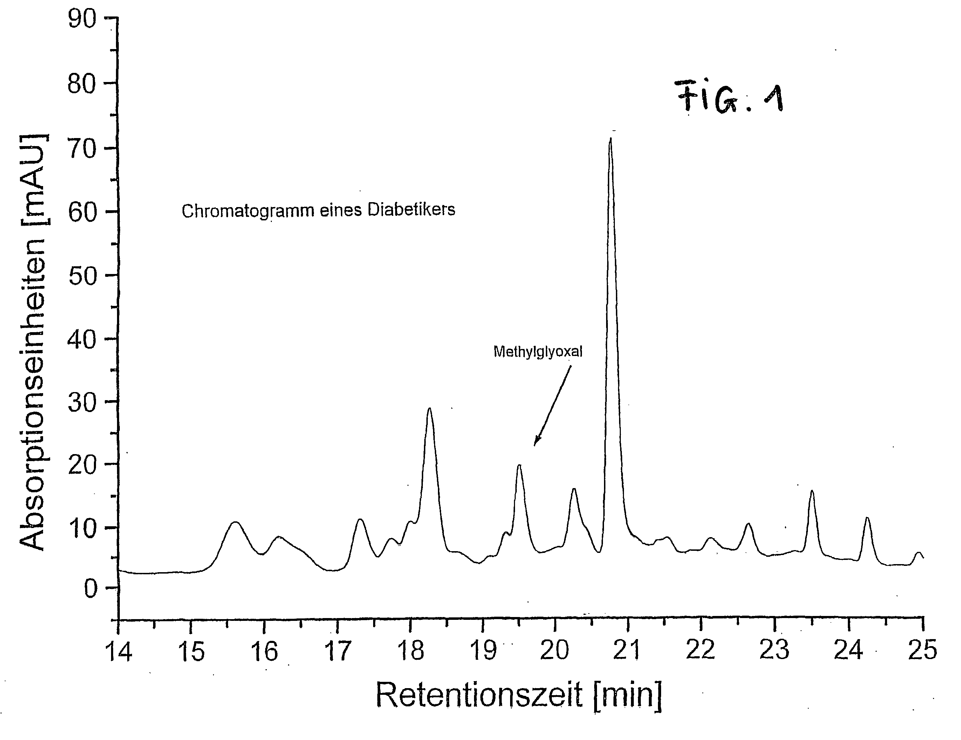

Zur Absicherung der Identifizierung der Substanzen wurde ein Teil des Eluates der Sammelkartusche unter den oben beschriebenen Bedingungen mit einem HPLC-System analysiert, das zusätzlich zum UV-Detektor über einen Massenspektrometrischen Detektor verfügte (Fig. 1). Dieser erlaubt die Identifizierung der Substanzen anhand ihrer Molekülmasse sowie durch den Zerfall des Molekül in charakteristische Molekülfragmente.

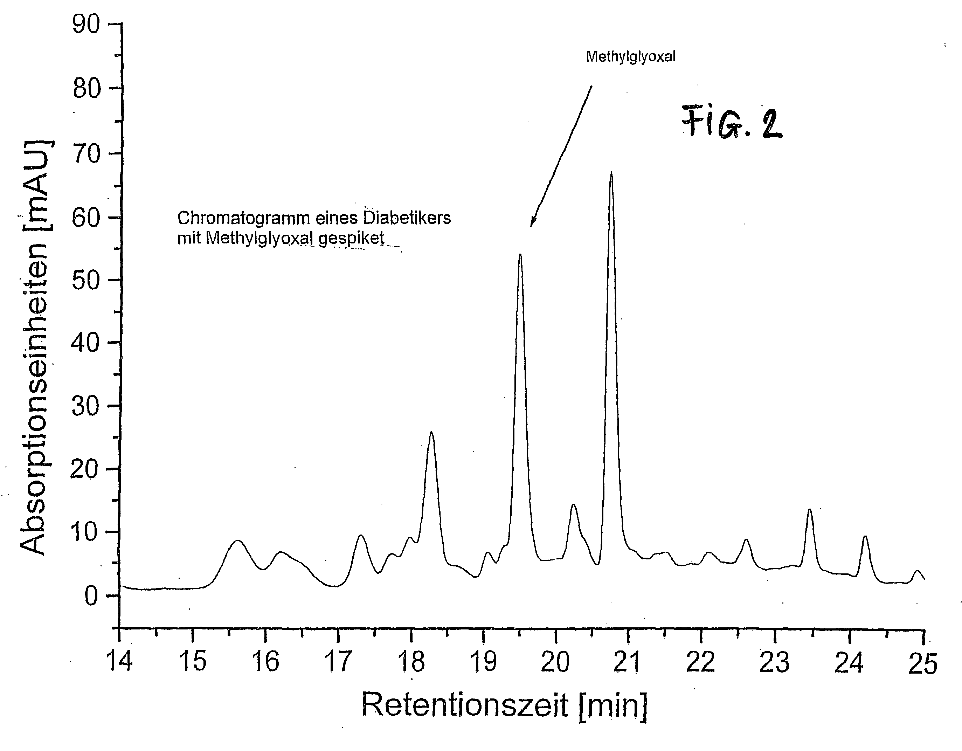

Einem weiteren Teil des Eluates wurden definierte Mengen der Derivate der α-Oxoaldehyde zugesetzt und die Lösung anschließend mittels HPLC und UV-Detektion analysiert (Prinzip der Standardaddition) (Fig. 2). In Abhängigkeit von der zugegebenen Menge an Derivat wird im Chromatogramm das entsprechende Signal der Verbindung erhöht und somit die Identifizierung bestätigt.

In Figur 1 ist unmittelbar der Peak für Aceton und der Peak für Methylglyoxal zu erkennen. Damit ist gezeigt, daß das Stoffwechselprodukt Methylglyoxal auch in der Atemluftprobe und damit nichtinvasiv nachgewiesen werden kann und aufgrund einer Stoffwechselspezifität unmittelbar auf das Vorhandensein von Methylglyoxal im Blutplasma der Patientin hinweist. Da Methylglyoxal nicht ubiquitär auftritt, sind Störungen der Messungen und Verfälschungen des Meßergebnisses durch das Umfeld der Patientin nicht zu erwarten.

Bei weiteren Vergleichsversuchen mit gesunden Vergleichsprobanden konnte bisher kein α-Oxoaldehyd in deren Atemluft nachgewiesen werden.

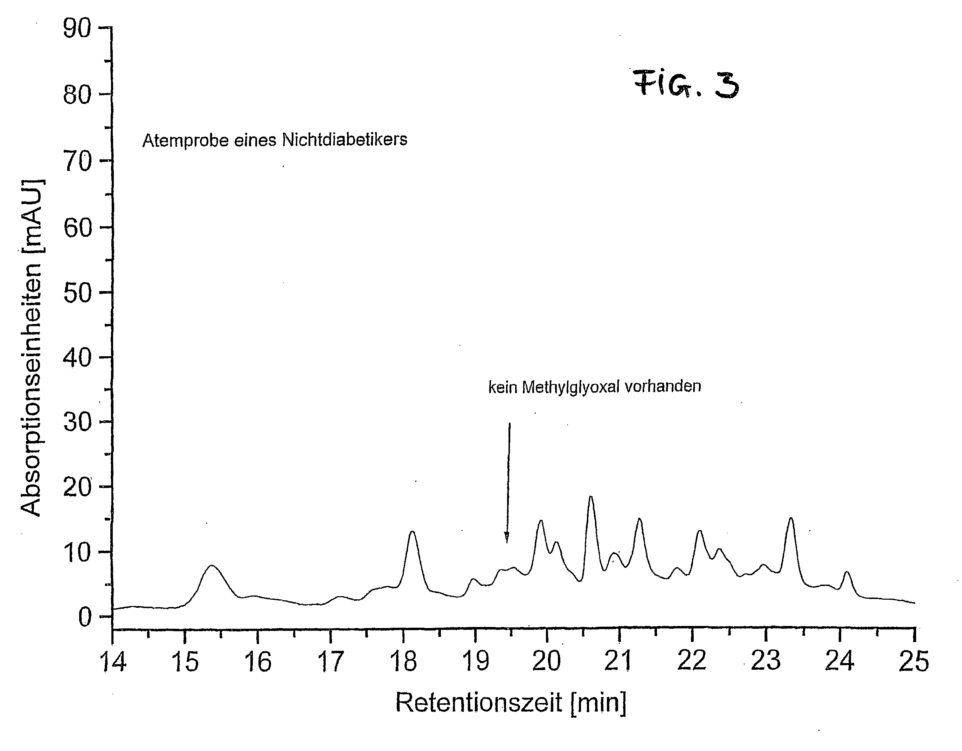

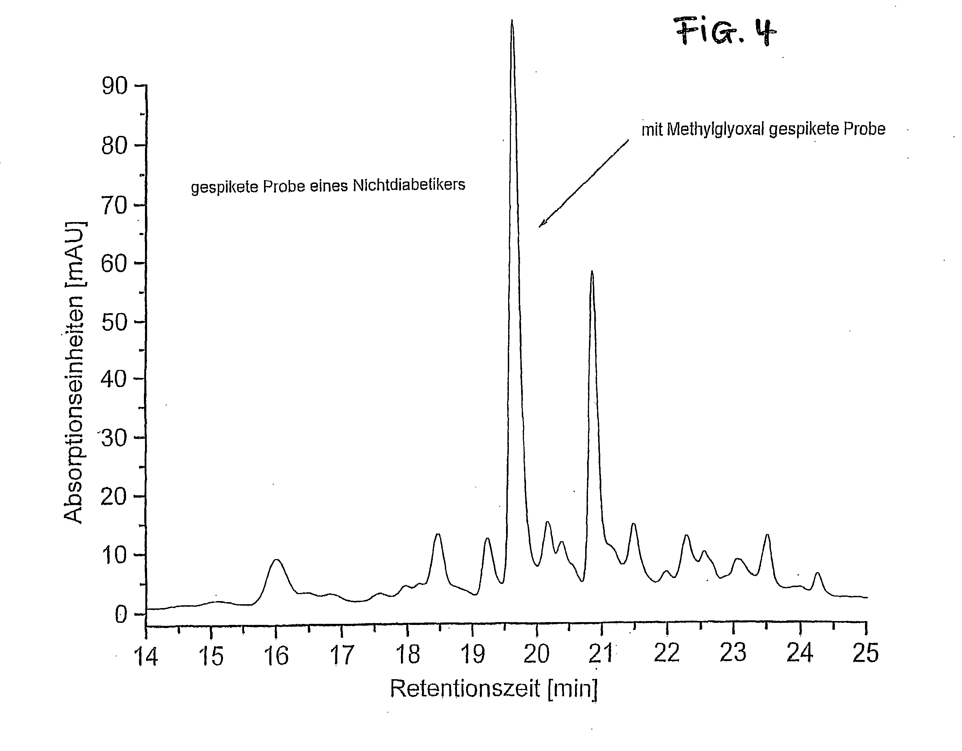

Beispielsweise wurde die Atemluftproben gesunder Vergleichsprobanden entsprechend dem oben beschriebenen Verfahren untersucht. Das resultierende Chromatogramm für einen gesunden Probanden zeigt Figur 3. Im Retentionszeitbereich der α-Oxoaldehyde ist kein auswertbares Signal zu erkennen. Nach Zusatz eines Methylglyoxal-Standards erscheint bei 19,50 Minuten im Chromatogramm ein Signal, wie in Figur 4 gezeigt ist.

## Patentansprüche

1. Verfahren zum Nachweis von α-Oxoaldehyden in Vollblut, Blutplasma und/oder Serum eines Patienten, wobei eine Atemluftprobe auf Vorhandensein mindestens eines α-Oxoaldehydes analysiert wird und aus dem Analyseergebnis auf das Vorhandensein von α-Oxoaldehyden im Vollblut, Blutplasma und/oder Serum des Patienten geschlossen. wird.

2. Verfahren nach Anspruch 1, wobei die Konzentration von α-Oxoaldehyden in der Atemluftprobe bestimmt wird und hieraus auf die Konzentration von α-Oxoaldehyden im Vollblut, Blutplasma und/oder Serum eines Patienten geschlossen wird.

3. Verfahren nach Anspruch 1 oder 2, wobei als α-Oxoaldehyd Methylglyoxal, Glyoxal und/oder 3-Deoxyglucuron nachgewiesen wird.

4. Verfahren nach einem der Ansprüche 1 bis 3, wobei die Atemluftprobe in einen Probenbeutel und/oder ein geeignetes Probenbehältnis eingeschlossen und/oder durch einen Impinger geleitet wird.

5. Verfahren nach einem der Ansprüche 1 bis 4, wobei die α-Oxoaldehyde in der Atemluftprobe mittels eines Derivatisierungsmittels derivatisiert und die entstehenden Derivate bestimmt werden.

6. Verfahren nach Anspruch 5, wobei die Derivate fluorometrisch, photometrisch und/oder chromatographisch nachgewiesen werden.

7. Verfahren nach einem der Ansprüche 1 bis 4, wobei die α-Oxoaldehyde in der Atemluftprobe bestimmt werden, indem die α-Oxoaldehyde elektrochemisch, spektrometrisch, enzymatisch und/oder immunchemisch nachgewiesen werden.

8. Verfahren nach einem der Ansprüche 1 bis 4, wobei die α-Oxoaldehyde in der Atemluftprobe massenspektrometrisch bestimmt werden.

9. Verfahren nach einem der Ansprüche 1 bis 4, wobei in der Atemluftprobe enthaltenes Methylglyoxal mit Glyoxalase zu Lactat umgesetzt wird und anschließend das Lactat mit einem Sensor nachgewiesen wird.

## Claims

1. Method of detecting alpha-oxoaldehydes in the whole blood, blood plasma and/or serum of a patient, in which a breath sample is analysed for the presence of at least one alpha-oxoaldehyde, and the presence of alpha-oxoaldehydes in the whole blood, blood plasma and/or serum of the patient is inferred from the analysis result.

2. Method as in Claim 1, in which the concentration of alpha-oxoaldehydes in the breath sample is established and, from this, the concentration of alpha-oxoaldehydes in the whole blood, blood plasma and/or serum of a patient is inferred.

3. Method as in Claim 1 or 2, in which methylglyoxal, glyoxal and/or 3-deoxyglucuron are detected as alpha-oxoaldehyde.

4. Method as in one of Claims 1 to 3, in which the breath sample is enclosed in a sample pouch and/or a suitable sample container, and/or is passed through an impinger.

5. Method as in one of Claims 1 to 4, in which the alpha-oxoaldehydes in the breath sample are derivatised by means of a derivatising medium and the resulting derivatives are determined.

6. Method as in Claim 5, in which the derivatives are detected by fluorometric, photometric and/or chromatographic means.

7. Method as in one of Claims 1 to 4, in which the alpha-oxoaldehydes in the breath sample are determined by detecting the alpha-oxoaldehydes by electrochemical, spectrometric, enzymic and/or immunochemical means.

8. Method as in one of Claims 1 to 4, in which the alpha-oxoaldehydes in the breath sample are determined by mass spectrometry.

9. Method as in one of Claims 1 to 4, in which methylglyoxal in the breath sample is converted, using glyoxalase, into lactate, and the lactate is then detected by means of a sensor.

## Revendications

1. Procédé de détection d'α-oxoaldéhydes dans du sang complet, du plasma sanguin et/ou du sérum d'un patient, dans lequel on analyse un échantillon d'air respiratoire quant à la présence d'au moins un α-oxoaldéhyde et on tire du résultat de l'analyse une conclusion sur la présence d'α-oxoaldéhydes dans le sang complet, le plasma sanguin et/ou le sérum du patient.

2. Procédé selon la revendication 1, dans lequel on détermine la concentration en α-oxoaldéhydes dans l'échantillon d'air respiratoire et on en tire une conclusion sur la concentration en α-oxoaldéhydes dans le sang complet, le plasma sanguin et/ou le sérum d'un patient.

3. Procédé selon la revendication 1 ou 2, dans lequel on détermine en tant qu'α-oxoaldéhyde, le méthylglyoxal, le glyoxal et/ou la 3-désoxyglucurone.

4. Procédé selon l'une quelconque des revendications 1 à 3, dans lequel l'échantillon d'air respiratoire est enfermé dans un sac à échantillon et/ou un récipient à échantillon approprié et/ou envoyé dans un impacteur

5. Procédé selon l'une quelconque des revendications 1 à 4, dans lequel les α-oxoaldéhydes dans l'échantillon d'air respiratoire sont transformés en dérivés au moyen d'un agent de dérivation et les dérivés résultants sont déterminés.

6. Procédé selon la revendication 5, dans lequel les dérivés sont détectés par fluorométrie, photométrie et/ou chromatographie.

7. Procédé selon l'une quelconque des revendications 1 à 4, dans lequel on détermine les α-oxoaldéhydes dans l'échantillon d'air respiratoire en détectant les α-oxoaldéhydes par voie électrochimique, spectrométrique, enzymatique et/ou immunochimique.

8. Procédé selon l'une quelconque des revendications 1 à 4, dans lequel on détermine les α-oxoaldéhydes dans l'échantillon d'air respiratoire par spectrométrie de masse.

9. Procédé selon l'une quelconque des revendications 1 à 4 dans lequel le méthylglyoxal contenu dans l'échantillon d'air respiratoire est converti en lactate à l'aide de glyoxalase et le lactate est ensuite étecté au moyen d'un capteur.
